# EUROPÄISCHE PATENTSCHRIFT

(11) **EP 3 316 829 B1**
(45) Veröffentlichungstag und Bekanntmachung des Hinweises auf die Patenterteilung: **05.08.2020**
(21) Anmeldenummer: 16734260.9
(22) Anmeldetag: 28.06.2016
(51) Int. Cl.: A61F 7/02, A61F 7/03

(54) **WÄRMEPADS MIT SPIRALFÖRMIGEN WÄRMEZELLEN**
HEAT PADS COMPRISING SPIRAL HEAT CELLS
COUSSINETS THERMIQUES AVEC CELLULES THERMIQUES EN FORME DE SPIRALE

(30) Priorität: 03.07.2015 DE 102015212494
(43) Veröffentlichungstag der Anmeldung: 09.05.2018
(60) Teilanmeldung: 20175726.7
(73) Patentinhaber: Beiersdorf AG, 20253 Hamburg (DE)
(72) Erfinder: WÖLLER, Karl-Heinz, 20257 Hamburg (DE); RÜCKER, Pia, 20357 Hamburg (DE); NIERLE, Jens, 21073 Hamburg (DE)
(86) Internationale Anmeldenummer: PCT/EP2016/064937
(87) Internationale Veröffentlichungsnummer: WO 2017/005536

(56) Entgegenhaltungen:
- DE-A1- 4 141 806
- GB-A- 2 505 059
- KR-A- 20120 035 092
- US-A- 311 552
- US-A- 2 246 238
- US-A1- 2008 262 536
- US-A1- 2010 280 581

## Beschreibung

Die Erfindung betrifft Wärmeprodukte zur Einmalbehandlung und/oder als Eigentherapie bei akuten, wiederkehrenden und/oder chronischen Schmerzzuständen aufweisend spiralförmig angeordnete wärmeerzeugende Materialien.

Wärmeprodukte zur Einmalbehandlung und/oder als Eigentherapie bei akuten, wiederkehrenden und/oder chronischen Schmerzzuständen in Muskeln und/oder Gelenken, bei Steifigkeitsgefühl, Nervenschmerzen, Rheumatismus, Menstruationsschmerzen etc. erfreuen sich seit den 1990er Jahren in Europa, Nordamerika und Australien zunehmender Beliebtheit bei den Anwendern und erzeugen einhergehend stetig wachsende Umsätze bei den Herstellern. Beispielhaft sei hier das HANSAPLAST Wärme-Therapie Pad genannt.

Der Ursprung von Wärmepads mit der Wärmeerzeugung durch exotherme Reaktion liegt im asiatischen Raum. Bis heute gilt für die Produkte der Japanese Industrial Standard (JIS) S 4100 "Disposible body warmers" von 1996 als weltweit einziger anerkannter Standard für die Definition der Messgrößen und Messmethoden von selbstwärmenden Produkten, auch wenn sich der Mindestanforderungen dieses Standards hinsichtlich Anwendungsdauer durch die Markt- bzw. Kundenanforderungen inzwischen als deutlich überholt erwiesen haben.

Im Stand der Technik sind wärmeerzeugende Stoffgemische seit Langem bekannt, beispielhaft sei hier WO2013054138 oder US2010/0280581 genannt.

Technologische Basis dieser Wärmeprodukte ist in der Regel die kontrollierte und exotherm verlaufende Oxidation von Eisenpulver oder feinverteilten Eisenspänen, die in einem Stoffgemisch enthalten sind, wobei das Stoffgemisch mindestens Kohlenstoffpulver, Wasser und ein Salz als Elektrolyt oder lonenbildner aufweist. Als weitere Bestandteile des wärmeerzeugenden Stoffgemisches können z.B. Holzkohlespäne, Feuchthaltemittel, Agglomerationshilfsstoffe, Bindemittel, Metallsalze, organisch oder anorganisch Füllstoffe und viel andere Substanzen mehr enthalten sein, um die gewünschten Endprodukteigenschaften einzustellen.

Diese dem Fachmann geläufigen Stoffgemische zur Abgabe von Wärme durch kontrollierte exotherme Oxidation, können für den Einsatz in einem Wärmepad als Pulver oder zu Granulaten, Agglomeraten, Perlen, Pellets oder Tabletten verdichtet eingesetzt werden. Zur Anwendung im Wärmepad sind die beschriebenen wärmeerzeugenden Stoffgemische üblicherweise in sogenannte "Wärmezellen" segmentiert eingearbeitet.

Diese Wärmezellen bilden sich üblicherweise dadurch, dass eine definierte Menge wärmeerzeugenden Stoffgemisches von zwei mit einander verbundenen Polymerfolien vollständig umschlossen ist, wobei mindestens eine Polymerfolie definiert sauerstoffdurchlässig sein muss.

Die Wärmezellen können auch die Form einer geschlossenen Röhre, eines Schlauches oder Beutels haben. Wärmeerzeugende Stoffgemische können auch auf Tiefziehfolien gegeben und dann eingesiegelt werden (z.B EP 1782787).

Im Wärmepad können diese Wärmezellen dann in unterschiedlichen Größen, unterschiedlicher Anzahl und unterschiedlichen Konfigurationen zwischen zwei laminatartig, z.B. durch Verklebung oder Verschmelzung, verbundene Trägersubstrate fixiert werden, wobei das Trägermaterial das an die sauerstoffdurchlässige Polymerfolie der Wärmezelle grenzt auch sauerstoffdurchlässig sein muss. Bei diesen Trägersubstraten kann es sich um Folien, Gewebe, Vliese, Gaze oder jedwedes andere für die Applikation auf dem menschlichen Körper geeignete Trägersubstrat handeln.

In einer Anwendungsform werden die Wärmepads auf der später hautzugewandten Seite selbstklebend ausgerüstet. Hierzu wird ein entsprechender hautfreundlicher Kleber auf das Trägersubstrat aufgetragen. Der Klebstoffauftrag kann vollflächig oder teilflächig, z.B. wellenförmig, punktuell, durchgehend oder unterbrochen gitterartig mit unterschiedlichen Gittergrößen und -formen erfolgen. Als Klebstoff sind dabei eine Vielzahl unterschiedlicher Klebstoffe aus dem Stand der Technik bekannt, z.B. Acrylat-, Synthesekautschuk- oder Silikonkleber.

Um den direkten Kontakt der Haut mit dem Klebstoff des Wärmepads zu vermeiden, z.B. bei besonders gegenüber Klebstoffen empfindlichen Anwendern, können die Wärmepads auch auf die Innenseite von hautnahen Bekleidungsteilen wie T-Shirts oder Unterhemden aufgeklebt werden. Das dabei zwangsweise vorhandene wärmeisolierende Luftpolster zwischen Haut und Wärmepad verringert jedoch eine gute Wärmeübertragung vom Wärmepad auf den Köper.

Es ist auch bekannt, die Wärmepads nicht selbstklebend auszurüsten, sondern zur Anwendung in vorgefertigte Taschen oder Aussparungen von eigens für diesen Zweck angefertigten Gürteln, Schlauchverbänden, Leibchen, Westen oder ähnlichen Fixierhilfen auf dem Körper zu befestigen. Auch die Fixierung von Wärmepads mittels Pflastern oder medizinischen Klebebändern (medical Tapes) ist bekannt.

Bei der Anwendung von Wärmepads als Wärmegürtel sind die entsprechenden Wärmezellen laminatartig zwischen einem hautabgewandten und einem hautzugewandten Trägersubstrat fixiert.

Da die Wärmezellen im Wärmepad bzw. Wärmegürtel durch Kontakt mit Luftsauerstoff aktiviert werden, müssen die Wärmepads direkt nach Herstellung in luftundurchlässige Verpackungen eingesiegelt werden. Erst vor der Anwendung wird dann das Wärmepad der Verpackung entnommen und der Sauerstoffkontakt leitet dann den exothermen Prozess (Oxidation des im wärmeerzeugenden Stoffgemisch enthaltenen Eisens) ein.

Gemäß JIS S 4100 soll die Wärmeerzeugung nach 30 min soweit fortgeschritten sein, dass eine Anwendungstemperatur auf der Haut zwischen 40 und 45 °C erreicht wird. Nachteilig ist bei dieser Art von Wärmepads dabei der Umstand, dass das vor der Anwendung pulverartige Stoffgemisch in den Wärmezellen während des Wärmeerzeugungsprozesses gesamtflächig miteinander zu einem steifen Festkörper verblockt (verklumpt, zusammensintert).

Die Wärmezellen sind damit nicht mehr geeignet bei einem sich bewegenden Anwender den verändernden Hautoberflächenkonturen zu folgen, es können dadurch sogar bestimmte Bewegungsabläufe gehemmt bzw. behindert werden. Aber auch bei weniger behinderten Bewegungsabläufen werden diese starren Wärmezellen von vielen Anwendern als störend empfunden.

Weiterhin können solcherart steife Wärmezellen dazu führen, das die Wärmepads bei und/oder nach Bewegungen des Anwenders keinen vollständigen Hautoberflächenkontakt mehr haben und somit nicht die volle heilwirksame Wärmeleistung des Produktes auf den Anwender übertragen wird.

Eine Möglichkeit die durch die 'aushärtenden' Wärmezellen bedingte nachteilige Versteifung von Wärmepads zu reduzieren wird z.B. in der DE 69729585 T2 offenbart. Hieraus weiß der Fachmann dass wenn er die bisher üblicherweise großflächig rechteckigen Wärmezellen durch kleinflächige, runde, ovale oder rechteckige Wärmezellen mit spezifischer geometrischer Anordnung zueinander ersetzt, über die Gesamtfläche des Wärmepads in mehreren Richtungen wärmezellenfreie Achsen entstehen die bei Bewegung des Anwenders als Gelenkachsen wirken können.

Nachteilig an dieser Lösung ist jedoch, dass auch diese kleineren Wärmezellen zu jeweils steifen Einzelzellen verblocken.

Die Gelenkachsen haben jedoch den Nachteil, dass der Bereich der Wärmezellen des Wärmepads durch steife bzw. halbstarre Materialien gestützt werden muss, um Verwindungen oder Faltenbildung während der Applikation zu verhindern.

Es gibt besondere Ausführungsformen von Wärmepads, für bestimmte Anwendungsfälle. Eine beliebte Form sind Wärmegürtel für die Anwendung im Lendenwirbelbereich, wie sie zum Beispiel in der US1996/0777830 offenbart werden.

Den Nachteilen des Standes der Technik abzuhelfen, insbesondere verbesserte und elastischere Wärmepads zur Verfügung zu stellen, war also die Aufgabe der Erfindung.

Wärmeprodukte gemäß JIS S 4100 bei denen die Wärmezellen enthaltende Grundfläche nicht vollflächig mit der zu behandelnden Haut verklebt ist, wie z.B. nur an 2 bis 3 Punkten verklebte Produkte analog ThermaCare® Wärmeauflagen oder S-O-S® Wärme-Umschläge, haben den Nachteil, dass der in der Regel mittig angebrachte und nicht klebende Bereich der Wärmezellen sich bei Bewegung des Anwenders von der Haut abhebt und sich eine wärmeisolierende Luftschicht zwischen Produkt und Haut ergibt. Da Luft ein sehr guter Wärmeisolator ist, kommt es hierdurch zu einer Verminderung der Heilwirkung der Wärmeprodukte.

Um die Wärmeabgabe zu verlängern bzw. um eine gleichmäßigere Wärmeabgabe zu erreichen, ist aus dem Stand der Technik bekannt (z.B. in US 2012/0150268 und JP 2005/137465), den wärmeerzeugenden Stoffgemischen Phase Changing Materials (PCMs) zuzusetzen.

PCMs sind Substanzen, die beim Schmelzen oder Erstarren bei einer bestimmten Temperatur große Mengen an Energie speichern bzw. freisetzen können. Das macht PCMs zu einem latenten Wärmespeicher und zu einem geeigneten Material um die Auswirkungen von möglichen lokalen Überhitzungen von Wärmezellen für die Anwendung am Menschen signifikant zu reduzieren. Wenn PCMs beim Erwärmen die Temperatur erreichen an dem sie die Phase wechseln, z.B. die Schmelztemperatur, absorbieren sie große Mengen an Wärmeenergie bei nahezu konstanter Temperatur bis alles Material geschmolzen ist. Wenn die Umgebungstemperatur wieder fällt erstarren die PCMs und geben die gespeicherte Energie wieder ab.

Als PCMs geeignete Materialien für die Anwendung in Wärmezellen sind z.B. Alkane mit 14 bis 30 Kohlenstoffatomen bzw. Gemische aus diesen Alkanen. Entsprechende Materialien und deren Verwendung werden z.B. in US 2012/0150268 offenbart.

Nachteilig an den beschriebenen PCMs ist, dass diese sich beim Schmelzen mit den restlichen Komponenten des wärmeerzeugenden Stoffgemisches einer Wärmezelle vereinigen und somit ebenfalls zu einer steifen Gesamtstruktur verblocken.

Auch der Einsatz von mikroverkapselten PCMs sind für Wärme- und Kühlpads aus dem Stand der Technik dem Fachmann bekannt (z.B. US 2003/0109910 und CA 2289971). Jedoch wird bei den bekannten Lösungen das mikroverkapselte PCM jeweils in Lösungen oder Gele eingebettet, die vor Anwendung in einer Mikrowelle oder einem Wasserbad erhitzt werden müssen. Wärmerzeugende Stoffgemische mit einem Anteil an mikroverkapselten PCMs, die für Wärmezellen geeignet sind, sind nicht bekannt. Spiralförmige Heizkissen sind aus DE4141806 bekannt.

Die Erfindung wird im unabhängigen Anspruch 1 definiert, bevorzugte Ausführungsformen werden in den abhängigen Ansprüchen beschrieben.

Es war überraschend und für den Fachmann nicht vorauszusehen, dass die Steifigkeit von "durchoxidierten" Wärmezellen dadurch deutlich verringert und der Tragekomfort für den Anwender 4 deutlich gesteigert werden kann, wenn die Wärmezellen nicht als flächige Einzelstrukturen wie Rechtecke, Kreise, Ellipsoide, Rhomboide etc. sondern als feingliedrige Spiralen ausgestaltet werden.

Erfindungsgemäß sind daher Wärmepads mit einer oder mehreren Wärmezellen, dadurch gekennzeichnet, das die Wärmezellen eine spiralförmige Gestalt aufweisen.

Auch war für den Fachmann nicht vorhersehbar, dass durch den Einsatz von mikroverkapselten PCMs eine Steifigkeitsverminderung erreicht werden kann, da diese sich nicht mit den restlichen Komponenten des wärmeerzeugenden Stoffgemisches vereinigen und dadurch die Beweglichkeit des Zellverbundes erhöhen.

Im Sinne der Erfindung ist daher auch, dass die erfindungsgemäßen Wärmepads ein oder mehrere Wärmezellen aufweisen, die wärmeerzeugende Stoffgemische enthalten, die mikroverkapselte PCMs aufweisen.

Kommerziell erhältlich sind eingekapselte PCMs z.B. unter dem Handelsnamen Lurapret® oder Micronal® bei der Firma BASF.

Erfindungsgemäße Wärmepads können zur direkten Fixierung auf dem zu behandelnden Körperareal selbstklebend beschichtet sein oder mittels zusätzlicher Fixierhilfen über dem zu behandelnden Körperareal angeordnet werden.

Spiralen haben das Potential Krafteinwirkung durch Zug aufgrund ihrer gebogenen Geometrie abzufedern. Bei einer Archimedischen Spirale maximal bis der gebogene Umfang zwischen zwei gegensätzlichen Krafteinwirkungspunkten zu einer gestreckten Geraden wird oder ein sprödes Material vorher bricht. Die absolute Länge des durch eine Kraft angegriffenen Spiralarms, und indirekt damit die Größe einer Spirale, bestimmt somit die Verringerung der Steifigkeit einer solcherart ausgefertigten Wärmezelle.

Im Gegensatz zu flächig ausgeprägten Wärmezellen bei denen eine möglichst geringe Größe und somit eine Vielzahl von separat angeordneten Wärmezellen vorteilhaft für eine geringe Steifigkeit des Gesamtprodukts ist, verringert sich die Steifigkeit der Endprodukte bei spiralförmig ausgeprägten Wärmezellen mit der Größe der Wärmezellen. Die Größe der Wärmezellen kann dabei durch den Kreisumfang als auch durch die Anzahl der Windungen der einzelnen Spiralarme bestimmt werden.

Die spiralartige Wärmezelle kann dabei in der Grundform als z.B. Archimedische, logarithmische, hyperbolische oder Fermatsche Spirale, Lituus-Spirale, Wurzelschnecke, Triskele oder Klothoide ausgebildet sein, oder aus einer oder mehreren beliebigen Kombinationsformen aller bekannten Spiralen.

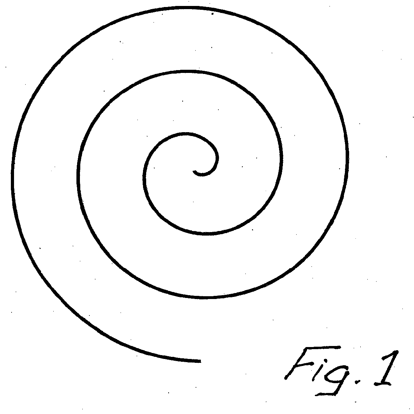

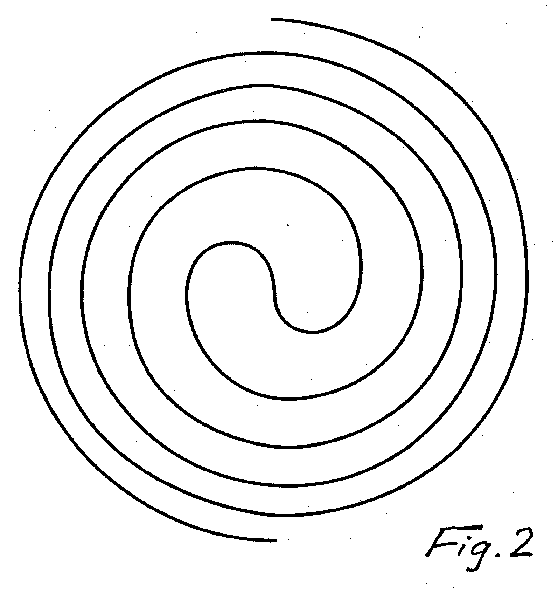

Insbesondere ist es von Vorteil, wenn eine erfindungsgemäße Wärmezelle nicht als Archimedische Spirale (Figur 1), sondern als Fermatsche Spirale (Figur 2) ausgebildet wird.

Die zweiarmigen Fermatschen Spiralen bieten durch die durchgängige Verbindung der Spiralarme über die gesamte Spiralfläche das maximale Potenzial zur Verringerung der Steifigkeit von Wärmezellen. Ein weiterer positiver Aspekt von Fermatschen Spiralen ist, das beide Spiralarme von der Mitte ausgehend am äußeren Rand der Gesamtstruktur enden und daher wiederum mit den äußeren Enden anderer, insbesondere bevorzugt Fermatscher, Spiralen verbunden werden können um die Steifigkeit der Gesamtstruktur weiter zu verringern.

Mehrarmige Fermatsche Spiralen haben zwar ein etwas geringeres Potenzial zur Verhinderung der Steifigkeit von Wärmezellen, bieten aber die Möglichkeit des Verbunds mit mehreren, insbesondere wiederum Fermatschen, Spiralen und somit einer weiteren Verringerung der Steifigkeit.

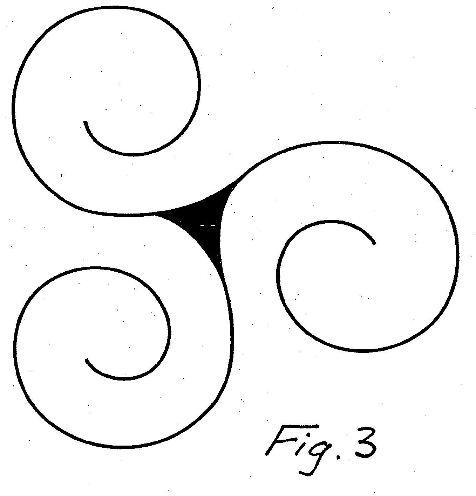

Besonders bevorzugt ist die Form einer oder mehrerer Triskelen oder Dreifach-Spiralen (Figur 3) um ein Netzwerk von zusammenhängenden Spiralen aufzuspannen.

Ein weiterer großer Vorteil und neuartig für die Ausprägung von Wärmezellen als Spiralen ist die Tatsache, dass Spiralen nicht nur Beweglichkeitspotenzial in x- und y-Achse haben, sondern zusätzlich auch in z-Achse senkrecht zur Ebene der Spirale. Dies ist für Anwender von entsprechend ausgearbeiteten Wärmeprodukten besonders relevant, wenn die Produkte über bei Bewegung aus dem Körper hervortretenden Partien, z. B. über Gelenken oder dem Schulterblatt, angewendet werden sollen.

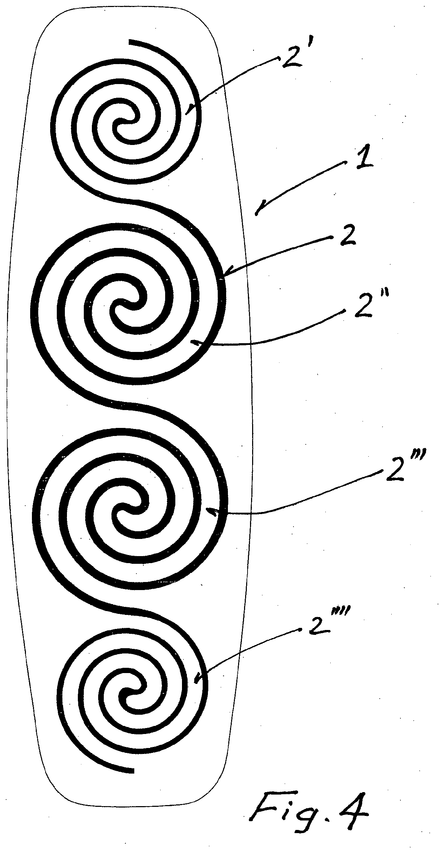

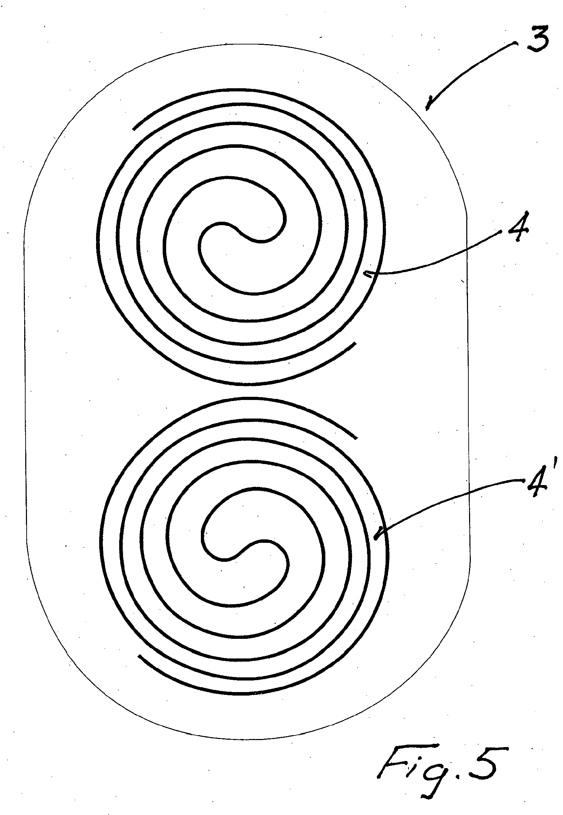

Ein erfindungsgemäßes selbstklebendes oder nicht selbstklebendes Wärmeprodukt analog JIS S 4100 kann eine spiralförmige Wärmezelle oder eine beliebige Pluralität von einzelnen (Figur 5) oder miteinander verbundenen (Figur 4) spiralförmigen Wärmezellen enthalten. Die spiralförmigen Wärmezellen müssen dabei nicht exakt kreisrund, sondern können auch oval, ellipsoid, quadratisch oder rechteckig-länglich ausgeführt sein. Insbesondere für großflächige Wärmeprodukte zur Behandlung im Lendenwirbelbereich des Rückens sind rechteckig-längliche Spiralformen vorteilhaft. Zur Erlangung einer rechteckig-länglichen Spiralform mit gleichmäßigem Abstand der Spiralarme voneinander können dabei die Spiralarme auf der x-Achse mit fließenden Übergängen deutlich breiter ausgeführt sein als jeweils auf der y-Achse.

Die Grundfläche einer erfindungsgemäßen Wärmezelle in Spiralform kann von 0,75 cm² bis 1300 cm² betragen, bevorzugt 3 cm² bis 620 cm², besonders bevorzugt 20 cm² bis 320 cm², ganz besonders bevorzugt 50 cm² bis 250 cm², wobei nur der vom wärmeerzeugenden Stoffgemisch bedeckte Bericht gemeint ist.

Die Breite eines Spiralarms kann von 0,1 cm bis 5 cm, bevorzugt 0,2 cm bis 3 cm, besonders bevorzugt 0,25 cm bis 2 cm betragen. Ebenso können die Breiten von Spiralarmen auf der x- und y-Achse einer flächig projizierten Spiralwärmezelle unterschiedlich ausgeprägt sein, ggfs. auch alternierend. Dies ist insbesondere bei großflächigen, rechteckig-länglichen Spiralen als Wärmezellen eine gute Ausgleichsmöglichkeit für ein homogenes Anwenderprodukt. Figur 4 zeigt eine Wärmezelle gebildet aus vier zusammenhängenden Spiralen, wobei die Arme der beiden äußeren Spiralen der Kette eine geringere Breite aufweisen, als die Arme der beiden innenstehenden Spiralen. Die Wärmeleistung der inneren Spiralen ist dadurch gegenüber den Äußeren erhöht.

Ein besonderer Vorteil der spiralförmigen Ausprägung der Wärmezellen ist darin zu sehen, dass vergleichsweise schmale Spiralarme im Gegensatz zu gesamthaft flächigen Wärmezellen einen deutlich geringeren Bruchwiderstand haben. Wird auf die Wärmezelle bei Anwendung durch Bewegung des Anwenders eine Kraft ausgeübt, brechen die Spiralarme aufgrund der geringeren Breite des Materialverbundes der oxidierten Mischung wesentlich leichter entlang der Bewegungsrichtung als flächige Wärmezellen. Dadurch kann sich jeweils genau entlang der jeweiligen maximalen Bewegungsbeanspruchung eine genau auf den Anwender abgestimmte Gelenklinie in der Wärmezelle ausbilden.

Ein durchschnittlicher Abstand der Spiralarme zueinander kann aufgrund der zugrundeliegenden Geometrien, besonders bei logarithmischen, hyperbolischen und insbesondere aufgrund des gemeinsamen Ursprungs der Spiralarme bei Fermatschen Spiralen, nicht gegeben werden. Bevorzugte Abstände zwischen den Windungen einzelner Spiralarme zueinander betragen innerhalb der Gesamtfläche einer Wärmespirale 0,1 cm bis 5 cm, besonders bevorzugt 0,2 cm bis 4 cm und ganz besonders bevorzugt 0,2 cm bis 3 cm.

Bei allen geometrischen Ausführungsformen von spiralförmigen Wärmezellen sind die Abstände in den Konfigurationen so zu wählen, dass die Wärmestrahlungen einzelner Spiralarme sich in der Haut des Anwenders möglichst überlappen um über die gesamte Behandlungsfläche ein homogenes Wärmegefühl zu ergeben.

Hinsichtlich ihrer Höhen bzw. Dicken können erfindungsgemäße spiralförmige Wärmezellen über die Spiralfläche deutlich differieren. So kann die Dicke einer Spirale zu ihrem Mittelpunkt hin gegenüber den Außenbereichen deutlich zunehmen als auch abnehmen, je nach gewünschten Anwendereigenschaften.

Die Höhe der Spiralfläche bzw. der Spiralarme einer Wärmezelle kann von 0,05 cm bis 1,5 cm, bevorzugt 0,05 cm bis 1,0 cm, besonders bevorzugt 0,05 cm bis 0,7 cm, ganz besonders bevorzugt 0,1 cm bis 0,5 cm variieren.

Der Füllgrad einer spiralförmigen Wärmezelle mit wärmeerzeugendem Stoffgemisch beträgt bevorzugt 50 % bis 100 % des maximalen Füllvolumens, besonders bevorzugt 70 % bis 100 %, ganz besonders bevorzug 90 % bis 100 %.

Das Gewicht einer erfindungsgemäßen spiralförmigen Wärmezelle beträgt bevorzugt 1 g bis 400 g, bevorzugt 2 g bis 300 g, besonders bevorzugt 4 g bis 250 g, ganz besonders bevorzugt 4,5 g bis 220 g.

Die erfindungsgemäßen Wärmepads weisen mehrere spiralförmige Wärmezellen auf, wobei die Wärmezellen auch verbunden oder ineinander verwoben sein können. Ganz oder teilweise selbstklebende Wärmepads sind bevorzugt rechteckig-länglich mit konisch zulaufenden Enden ausgeführt. Die Abmessungen des Wärmepads sind stark abhängig vom Einsatzort. Wärmepads die zum Beispiel für die Wärmebehandlung des Rückens vorgesehen sind, können bis zu 40 cm lang und 20 cm breit sein. Universeller einsetzbare Wärmepads weisen bevorzugt eine Länge zwischen 20 cm und 30 cm und einer Breite zwischen 10 cm und 15 cm auf. Es kann von Vorteil sein, Wärmepads in verschiedenen Größen, abgestimmt auf unterschiedliche Körpergrößen, für die gleichen Applikationsort anzubieten.

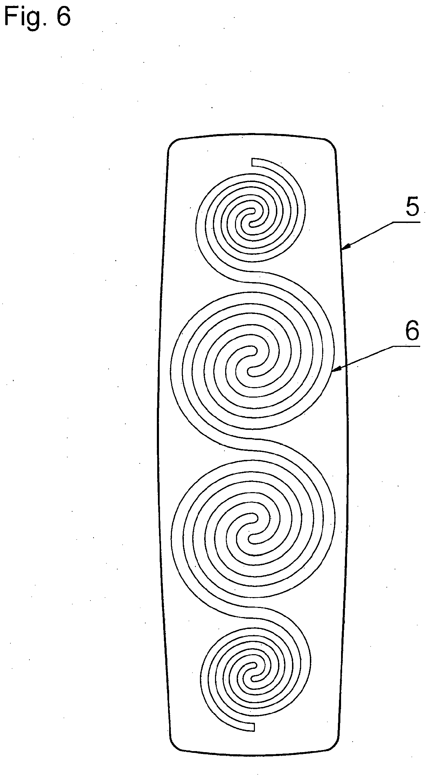

Besonders bevorzugt enthalten diese Wärmepads vier in Längsrichtung hintereinander angeordnete, ganz besonders bevorzugt miteinander verbundene Fermatsche Wärmezellen in identischer oder unterschiedlich Konfiguration hinsichtlich Größe, Form, Länge, Breite, Gewicht, sowie Länge und Abstand der Spiralarme untereinander. (Figur 6)

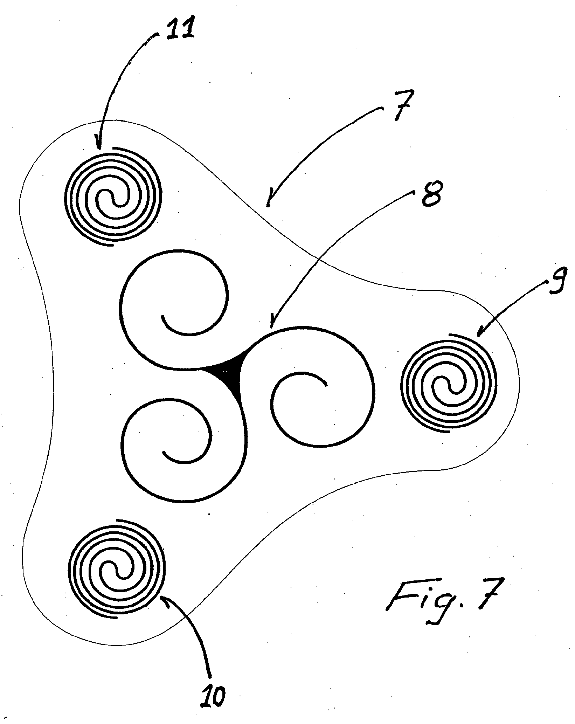

In einer weiteren erfindungsgemäßen Ausprägung sind dieserart Wärmepads in Form eines an den Seiten konkav eingeschnittenen und an den Ecken abgerundeten Dreiecks gefertigt. Figur 7 zeigt beispielshaft ein 'dreieckiges' Wärmepad mit vier separaten Wärmezellen, wobei im Zentrum eine triskelenförmige Wärmezelle sitzt, die von drei Wärmezellen in der Geometrie von Fermatschen Spiralen umgeben ist.

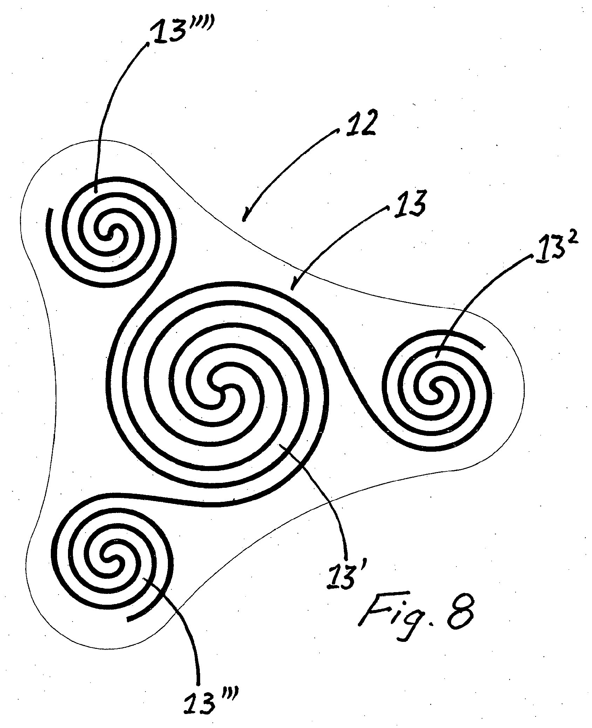

Bevorzugt enthalten erfindungsgemäße Wärmepads zentral mittig eine Wärmezelle mit Geometrie einer dreiarmige Fermatsche Spirale, welche am Endpunkt eines jeden Spiralarms mit je einer weiteren, in Richtung der Dreiecksspitzen zentrierten Wärmezelle in Form einer Fermatschen Spirale verbunden ist (Figur 8).

Erfindungsgemäße Wärmepads in Form eines Wärmegürtels enthalten bevorzugt 2 bis 8, besonders bevorzugt 2 bis 4, gegebenenfalls teilweise über die Spiralarme miteinander verbundene spiralförmige Wärmezellen. Die Wärmezellen sind dabei bevorzugt über eine wärmeabgebende Grundfläche von kleiner 25 cm mal 35 cm verteilt und können sowohl nebeneinander als auch übereinander angeordnet sein.

Bei einer weiteren Form eines wiederverwertbaren Wärmegürtels, werden ein oder mehrere nichtklebende Wärmepads in passend vorgefertigten Taschen platziert.

Der Wärmegürtel weist beidseitig Gurtelemente auf, welche aus einer Vielzahl dem Fachmann geläufigen Materialien und Formen und unterschiedlicher Elastizität bestehen können und an den Längsachsen in einem gemeinsamen Verschlusssystem enden. Bevorzugt besteht das gemeinsame Verschlusssystem aus waagerecht oder senkrecht gegeneinander aufgebrachten Teilen von Klett-, Klebe-, Hakenverschlüssen oder (Druck-) Knöpfen.

Das Material der Taschen zur Aufnahme der Wärmepads muss ausreichend dehnbar und elastisch sein, um einen sicheren Halt der Wärmepads zu gewährleisten.

In einem erfindungsgemäßen Wärmepad können spiralförmige Wärmezellen bevorzugt laminatartig zwischen längselastische, besonders bevorzug bi-elastische, Trägermaterialien eingebunden sein. Bei Einbindung zwischen nur längselastischen Trägermaterialien ist für eine maximale Reduzierung der Steifigkeit des Endprodukts darauf zu achten, dass die spiralförmige(n) Wärmezelle(n) mit ihrem maximalen Durchmesser in Richtung der Elastizität der Trägermaterialien angeordnet wird/werden.

Geeignete längs- oder bi-elastische Trägermaterialien sind in vielfältiger Weise kommerziell erhältlich. Längselastisch z.B. als Elastik-Gewebe der Fa. Kümpers, Rheine, Deutschland, einem Gewebe aus Baumwolle 4 % Lycra enthaltend, mit einem Flächengewicht von rund 180 g/m² und einer Dehnbarkeit auf über 200 % seiner Ausgangslänge. Oder z.B. Artikel 016 der Fa. KOB, Wolfstein, Deutschland, einem Gewebe bestehend aus 70 % Viskose und 30 % Polyamid mit einer Dehnbarkeit von 60 %.

Bielastische Gewebe sind ebenfalls von der Fa. KOB erhältlich, z.B. Artikel 023 aus 100 % Baumwolle mit einer Längsdehnbarkeit von 85 % und einer Querdehnbarkeit von 40 %, oder Artikel 053 aus einem 100 % PET-Gewebe mit einer Längsdehnbarkeit von 25 - 40 % und einer Querdehnbarkeit von > 40 %.

Weitere gut geeignete bielastische Materialien sind auch z.B. von der Fa. Innovatec, Troisdorf, Deutschland, erhältlich, z.B. Thermoplastische Polyurethane (TPU) mit einem Flächengewicht von 75 g/m² und einer Längsdehnbarkeit von 300 % und einer Querdehnbarkeit von 330 %.

Geeignete Trägermaterialien für erfindungsgemäße Wärmezellen können auf der hautabgewandten Seite auch vorteilhaft wärmeisolierend ausgerüstet sein. Durch diese, zumindest teilweise, Wärmeisolierung wird Abgabe von Wärme in den umgebenden Raum reduziert, dadurch können wärmerzeugende Materialien in den Zellen eingespart und das Gesamtgewicht des Endprodukts reduziert werden. Die Wärmeisolierung der Träger kann auf vielfältige Weise erzeugt werden, z.B. durch Metallfolienbeschichtungen oder auch durch Einarbeitung von natürlichen Resten aus der Kaffeeschalenaufarbeitung. Produkte unter Verwendung der letztgenannten Technologie sind z.B. unter der Bezeichnung NILIT® Heat kommerziell erhältlich.

Naturgegeben hat jeder Anwender von Wärmepads ein eigenes subjektives Empfinden der jeweils abgegebenen Wärmemenge. Der in der JIS S 4100 angegebene Temperaturbereich für diese Produkte kann von unterschiedlichen Anwender sowohl als gerade richtig, oder auch, mit einer Vielzahl an Zwischennuancen, als auch zu kalt oder zu heiß empfunden werden. Eine Möglichkeit hierbei für den individuellen Anwender Abhilfe zu schaffen besteht darin, die hautabgewandte Seite des Wärmezellenverbundes erfindungsgemäßer Produkte mit nur leicht haftenden Materialien unterschiedlicher Sauerstoffdurchlässigkeit zu versehen, bevorzugt mehrere Materialien mit von innen nach außen sich reduzierender Sauerstoffdurchlässigkeit in Laminatform. Ist dem Anwender eines solchen Produkts die Wärmeleistung nicht ausreichen genug kann er die äußere Materialschicht entfernen und mehr Sauerstoff kann den exothermen Prozess innerhalb einer Wärmezelle erreichen. Dadurch erhöhen sich die Reaktionsgeschwindigkeit und somit die resultierende Temperatur, allerdings bei entsprechender Reduktion der möglichen Gesamtnutzungsdauer des Wärmeprodukts. Durch Entfernen weiterer Materialschichten kann dieser Prozess entsprechend weiter individuell durch den Anwender beeinflusst werden.

Das beschriebene Prinzip ist auch umgekehrt anwendbar, in dem man dem Wärmeprodukt entsprechend haftende bedingt sauerstoffdurchlässige Materialschichten beigibt die vom Anwender je nach persönlicher Wohlfühltemperatur zusätzlich auf der äußeren Seite des Wärmeprodukts angebracht werden um die Sauerstoffzufuhr zu reduzieren, was wiederum zu einer reduzierten Gesamttemperatur und zu einer verlängerten Anwendungsdauer führt.

Beide oben ausgeführten Prinzipien sind auch mit Materialien identischer Sauerstoffdurchlässigkeit ausführbar, herbei kommen dann lediglich additive bzw. subtraktive Mechanismen der Reaktionsverlaufssteuerung zum Tragen.

Eine weitere vorteilhafte Möglichkeit zur besseren Nutzung bzw. Abgabe der Wärmeenergie erfindungsgemäßer Zellen besteht in der Zugabe von Phase Changing Materials (PCMs) zur den Trägermaterialien, besonders bevorzugt aber direkt als Komponente des exothermen Stoffgemisches.

Besonders bevorzugt zum Einsatz in exothermen Wärmemischungen in erfindungsgemäßer Spiralform sind eingekapselte PCMs deren Kapseldurchmesser kleiner ist als die Dicke der Spiralen und vorteilhaft 0,5 bis 1,5 mm beträgt. Da die Kapsel während der Temperaturübergänge der PCMs ihre äußere Form behalten gehen sie keinen Verbund mit der reagierenden und langsam durchhärtenden Wärmemischung ein und können somit aufgrund ihrer Größe bei Kraftbeaufschlagung des Anwenders durch Bewegung als inhärente Gelenke bzw. Sollbruchstelle in den Wärmespiralen dienen und dadurch für eine deutlich verringerte Steifigkeit bei Anwendung am Körper sorgen.

Der Nachteil des schlechten Wärmeüberganges bei nicht vollflächig verklebten Wärmepads kann verringert werden, indem auf der hautzugewandten Seite des Wärmepads im Bereich der Wärmezellen eine linsenförmige, halbkonvexe Schicht aus wärmeleitenden Polymeren aufgebracht wird. Diese linsenförmige Schicht kann in der Flächenausdehnung sowohl kreisrund, wie auch ellipsoid oder oval oder unregelmäßig ausgeprägt sein.

Bevorzugt werden hierbei Polymerschichten aus Silikon, denn Silikone haben sehr gute Elastizitäts- und Wärmeleiteigenschaften (z.B. Silikonharze bei RT von 0,15 bis 0,32 W/mK), insbesondere aber auch ein vergleichsweise gutes Kompressionsverhalten von 15 % bis 30 %. Linsenförmige Silikonschichten mit einer Dicke von 0,01 cm bis 2,5 cm und einem Durchmesser von maximal 2 cm bis 20 cm sind daher gut geeignet, den vollflächigen Hautkontakt der exothermen Wärmezellen zur optimalen Wärmeübertragung auch bei Bewegung des Anwenders zu gewährleisten. Besonders bevorzugt wird diese Schicht aus Silikonpolymeren mit einer Shore-Härte kleiner oder gleich 50 Shore A gefertigt.

Erfindungsgemäße Wärmepads unter Verwendung spiralförmiger Wärmezellen können mit Wärmeanzeigen versehen sein. Da sich das subjektive Wärmeempfinden des Anwenders in der Regel mit zunehmender Anwendungsdauer durch Gewöhnungseffekte verringern kann und das Wärmeprodukt dadurch bereits vor dem Ende der indizierten Behandlungszeit vom Anwender entfernt wird, ist eine optische Kontrolle der Temperatur von Vorteil. Entsprechende Indikatoren die durch chemische Farbreaktoren die Temperatur visualisieren können, sind z.B. aus US 2009/0149925 dem Fachmann bekannt.

Bei Wärmegürteln zur Mehrfachverwendung, bei denen die Wärmezellen enthaltenden Wärmepads vor der Anwendung jeweils erneuert werden, kann es vorteilhaft sein die Wärmeindikatoren in den nicht zu erneuernden Gürtel zu inkorporieren.

Vorteilhaft und im Sinne der Erfindung ist es, die erfindungsgemäßen Wärmepads mit Wirkstoffen zur Unterstützung der Therapie auszurüsten. Diese Wirkstoffe können dabei auf der hautzugewandten Seite der Produkte, inkorporiert in einem entsprechenden Depot, bis zur Anwendung vorgehalten werden, oder auch, bei selbstklebend ausgerüsteten Wärmepads, in die Klebmatrix eingearbeitet sein (sogenannte monolithische Systeme).

Erfindungsgemäße Wärmepads können z.B. auch mit hyperämisierenden Wirkstoffen, etwa Antiphlogistika und/oder Analgetika, wie natürliche Wirkstoffe des Cayenne-Pfeffers oder synthetische Wirkstoffe wie Nonivamid, Nicotinsäurederivate, bevorzugt Bencylnicotinat oder Propylnicotinat, ausgerüstet werden. Vorteilhaft sind Capsaicin ([8-Methyl-trans-6-nonensäure-(4-hydroxy-3- methoxybenzylamid)], Nonivamid, Nicotinsäurebenzylester oder Benzylnicotinat.

Ebenso sind nichtsteroidale Antirheumatika als Wirkstoffe geeignet, wie z.B. Glykolsalicylat, Flufenaminsäure, Ibuprofen, Etofenamat, Ketoprofen, Piroxicam, Indomethacin. Ebenfalls gut geeignet sind Antiphlogistika, wie Acetylsalicylsäure, oder Antipuriginosa, wie z.B. Polidocanol, Isoprenalin, Crotamiton, oder Lokalanästhetika, wie z.B. Lidocain, Benzocain.

Vorteilhaft ist es erfindungsgemäße Wärmepads mit Wirkstoffen zu dotieren, die den Zustand der Haut positiv beeinflussen. Diese Wirkstoffe können nicht nur zu einer besseren Hautverträglichkeit der selbstklebenden Wärmepads führen, sondern auch aktiv das äußere Erscheinungsbild der Haut z.B. bei Falten, Narben oder Cellulite verbessern. Als besonders bevorzugte Wirkstoffe gelten dabei Biochinone, insbesondere Ubichinon Q10, Kreatin, Kreatinin, Carnitin, Acetylcarnitin, Biotin, Isoflavon und Isoflavonoide, Genistein, Arctiin, Cardiolipin, Liponsäure, Anti Freezing Proteine, Hopfen- und Hopfen-Malz-Extrakte, und/oder die Restrukturierung des Bindegewebes fördernde Stoffe, ebenso wie Isoflavonoide sowie Isoflavonoid-haltige Pflanzenextrakte wie z.B. Soja- und Klee-Extrakte. Auch können Wirkstoffe zur Unterstützung der Hautfunktionen bei trockener Haut, wie beispielsweise Vitamin C, Biotin, Carnitin, Kreatin, Kreatinin, Propionsäure, Glycerin, Grüntee-Extrakte und Harnstoff.

Als Wirkstoffe können des Weiteren zu einer unterstützenden Aromatherapie ätherische Öle eingesetzt werden, z.B. bei der Anwendung erfindungsgemäßer Wärmepads bei Menstruationsbeschwerden. Die ätherischen Öle können dabei nicht nur in das hautzugewandte Trägersubstrat eingearbeitet sein, sondern insbesondere auch in das hautabgewandte Trägersubstrat. Besonders bevorzugt liegen die Wirkstoffe in verkapselter Form vor.

Unter ätherischen Ölen sind aus Pflanzen gewonnene Konzentrate zu verstehen, die als natürliche Rohstoffe hauptsächlich in der Parfüm- und Lebensmittelindustrie eingesetzt werden und die mehr oder weniger aus flüchtigen Verbindungen bestehen. Als Beispiele für diese Verbindungen können 1,8-Cineol, Limonen, Menthol, Borneol und Kampfer genannt werden. Oft wird der Begriff ätherische Öle für die noch in den Pflanzen enthaltenen flüchtigen Inhaltsstoffe verwendet. Im eigentlichen Sinn versteht man aber unter ätherischen Ölen Gemische aus flüchtigen Komponenten, die durch Wasserdampfdestillation aus pflanzlichen Rohstoffen hergestellt werden. Ätherische Öle bestehen ausschließlich aus flüchtigen Komponenten, deren Siedepunkte in der Regel zwischen 150 und 300 °C liegen. Sie enthalten überwiegend Kohlenwasserstoffe oder monofunktionelle Verbindungen wie Aldehyde, Alkohole, Ester, Ether und Ketone. Stammverbindungen sind Mono- und Sesquiterpene, Phenylpropan-Derivate und längerkettige aliphatische Verbindungen.

Bei manchen ätherischen Ölen dominiert ein Inhaltsstoff, zum Beispiel Eugenol in Nelkenöl mit mehr als 85%, andere ätherische Öle stellen hingegen komplex zusammengesetzte Mischungen der einzelnen Bestandteile dar. Oft werden die organoleptische Eigenschaften nicht von den Hauptkomponenten, sondern von Neben- oder Spurenbestandteilen geprägt, wie zum Beispiel von den 1,3,5-Undecatrienen und Pyrazinen im Galbanum-Öl. Bei vielen der kommerziell bedeutenden ätherischen Öle geht die Zahl der identifizierten Komponenten in die Hunderte. Sehr viele Inhaltsstoffe sind chiral, wobei sehr oft ein Enantiomer überwiegt oder ausschließlich vorhanden ist, wie zum Beispiel (-)-Menthol im Pfefferminzöl oder (-)-Linalylacetat im Lavendelöl.

Als bevorzugte ätherische Öle können Oleum Eucalypti, Oleum Menthae piperitae, Oleum camphoratum, Oleum Rosmarini, Oleum Thymi, Oleum Pini sibricum und Oleum Pini silverstris sowie die Terpene 1,8-Cineol und Levomethanol genannt werden.

### Figuren:

Figur 1 zeigt eine Archimedische Spirale. Sie entsteht, wenn bei einer Drehbewegung der Radius proportional zum Drehwinkel wächst.
Figur 2 zeigt eine Fermatsche Spirale
Figur 3 zeigt eine Triskele
Figur 4 zeigt beispielshaft ein Wärmepad 1, welches eine Wärmezelle 2 aufweist, wobei die Wärmezelle die Gestalt von vier miteinander verbundenen Fermatschen Spiralen 2', 2", 23"' und 2'''' aufweist.
Figur 5 zeigt beispielshaft ein Wärmepad 3, welches zwei Wärmezellen 4, 4' aufweist, wobei die Wärmezellen jeweils die Gestalt von Fermatschen Spiralen aufweisen.
Figur 6 zeigt beispielshaft ein rechteckig-länglich mit konisch zulaufenden Enden gestaltetes Wärmepad 5, welches eine Wärmezelle 6 aufweist, wobei die Wärmezelle die Gestalt von vier miteinander verbundenen Fermatschen Spiralen aufweist.
Figur 7 zeigt beispielshaft ein 'dreieckiges' Wärmepad 7 mit vier separaten Wärmezellen 8,9,10,11, wobei im Zentrum eine triskelenförmige Wärmezelle 8 sitzt, die von drei Wärmezellen 9,10,11 in der Geometrie von Fermatschen Spiralen umgeben ist.
Figur 8 zeigt beispielshaft ein 'dreieckiges' Wärmepad 12 mit einer Wärmezelle 13, wobei die Wärmezelle im Zentrum eine Geometrie einer dreiarmige Fermatsche Spirale 13' hat, welche am Endpunkt eines jeden Spiralarms mit je einer weiteren, in Richtung der Dreiecksspitzen zentrierten Wärmezellenabschnitt 13", 13"', 13"" in Form einer Fermatschen Spirale übergeht.

## Patentansprüche

1. Wärmepad mit mehreren Wärmezellen, wobei die Wärmezellen eine spiralförmige Gestalt aufweisen und wobei die Wärmezellen ein wärmeerzeugendes Substanzgemisch aufweisen, **dadurch gekennzeichnet, dass** das wärmeerzeugende Stoffgemisch neben anderen Komponenten mindestens Eisenpulver und Kohlenstoffpulver enthält, wobei das Wärmepad die Form eines an den Seiten konkav eingeschnittenen und an den Ecken abgerundeten Dreiecks aufweist und wobei das Wärmepad mindestens vier spiralförmige Wärmezellen aufweist oder dass das Wärmepad eine rechteckig-längliche Form aufweist und wobei das Wärmepad mindestens zwei in Längsrichtung hintereinander angeordnete spiralförmige Wärmezellen aufweist und wobei die spiralförmigen Wärmezellen als miteinander verbundene Fermatsche Spiralen gestaltet sind.

2. Wärmepad nach Anspruch 1, **dadurch gekennzeichnet, dass** mindestens eine Wärmezelle die Form einer Archimedischen Spirale, einer Fermatschen Spirale, einer Triskele oder einer Vielfach-Spirale aufweist.

3. Wärmepad nach Anspruch 2, **dadurch gekennzeichnet, dass** die Spirale eine ovale oder eckige Form aufweist.

4. Wärmepad nach mindestens einem der vorhergehenden Ansprüche, **dadurch gekennzeichnet, dass** die Oberfläche zumindest teilweise mit eine Klebeschicht ausgerüstet ist.

5. Wärmepad nach mindestens einem der vorhergehenden Ansprüche, **dadurch gekennzeichnet, dass** die Wärmezelle ein wärmeerzeugendes Substanzgemisch aufweist, wobei eine definierte Menge wärmeerzeugenden Stoffgemisches von zwei mit einander verbundenen Polymerfolien vollständig umschlossen ist, wobei mindestens eine Polymerfolie sauerstoffdurchlässig ist.

6. Wärmepad nach mindestens einem der vorhergehenden Ansprüche, **dadurch gekennzeichnet, dass** die Wärmezelle ein wärmeerzeugendes Substanzgemisch aufweist, wobei eine definierte Menge wärmeerzeugenden Stoffgemisches in einer Röhre oder einem Schlauch eingeschlossen ist, wobei die Röhre oder der Schlauch aus einem sauerstoffdurchlässigen Material besteht.

7. Wärmegürtel, aufweisend mindestens ein Wärmepad nach mindestens einem der vorhergehenden Ansprüche, **dadurch gekennzeichnet, dass** das Wärmepad mittels an den Längsachsen angebrachten Gurtelementen, die in einem korrespondierendem Verschlusssystem enden, eine schlaufenförmige bzw. körpereinhüllende Verbindung ermöglicht.

8. Wärmegürtel nach Anspruch 8, **dadurch gekennzeichnet, dass** die Wärmezellen austauschbar sind.

9. Wärmepad nach mindestens einem der vorhergehenden Ansprüche, **dadurch gekennzeichnet, dass** die wärmeerzeugende Stoffgemische in mindestens einer Wärmezelle, mikroverkapselte PCMs aufweisen.

10. Wärmepad nach mindestens einem der vorhergehenden Ansprüche, **dadurch gekennzeichnet, dass** auf der hautzugewandten Seite des Wärmepads im Bereich der Wärmezellen eine linsenförmige, halbkonvexe Schicht aus wärmeleitenden Polymeren aufgebracht ist.

## Claims

1. Heat pad having two or more heat cells, wherein the heat cells have a spiral shape and wherein the heat cells include a heat-generating substance mixture, **characterized in that** the heat-generating material mixture comprises, as well as other components, at least iron powder and carbon powder, wherein the heat pad takes the form of a triangle having concave recesses in the sides and which is rounded off at the corners and wherein the heat pad has at least four heat cells in spiral form, or **in that** the heat pad has a rectangular elongated form and wherein the heat pad has at least two cells in spiral form arranged successively in longitudinal direction and wherein the heat cells in spiral form are configured as Fermat spirals bonded to one another.

2. Heat pad according to Claim 1, **characterized in that** at least one heat cell has the shape of an Archimedean spiral, a Fermat spiral, a triskelion or a multiple spiral.

3. Heat pad according to Claim 2, **characterized in that** the spiral has an oval or angular shape.

4. Heat pad according to at least one of the preceding claims, **characterized in that** the surface has at least partly been modified with an adhesive layer.

5. Heat pad according to at least one of the preceding claims, **characterized in that** the heat cell includes a heat-generating substance mixture wherein a defined amount of heat-generating material mixture is fully surrounded by two polymer films bonded to one another, at least one polymer film being oxygen-permeable.

6. Heat pad according to at least one of the preceding claims, **characterized in that** the heat cell includes a heat-generating substance mixture wherein a defined amount of heat-generating material mixture is enclosed in a tube or a hose, the tube or hose consisting of an oxygen-permeable material.

7. Heat belt including at least one heat pad according to at least one of the preceding claims, **characterized in that** the heat pad enables joining in the form of a loop, enwrapping the body, by means of belt elements mounted on the longitudinal axes that end in a corresponding fastener system.

8. Heat belt according to Claim 8, **characterized in that** the heat cells are exchangeable.

9. Heat pad according to at least one of the preceding claims, **characterized in that** the heat-generating material mixtures include, in at least one heat cell, microencapsulated PCMs.

10. Heat pad according to at least one of the preceding claims, **characterized in that** a lenticular, semiconvex layer of heat-conducting polymers has been applied to the skin-facing side of the heat pad in the region of the heat cells.

## Revendications

1. Coussinet chauffant comprenant plusieurs cellules chauffantes, les cellules chauffantes ayant une forme en spirale et les cellules chauffantes comportant un mélange de substances générant de la chaleur, **caractérisé en ce que** le mélange de substances générant de la chaleur contient, en plus d'autres composants, au moins de la poudre de fer et de la poudre de carbone, le coussinet chauffant ayant la forme d'un triangle découpé concave sur les côtés et arrondi aux coins, et le coussinet chauffant comportant au moins quatre cellules chauffantes en spirale, ou **en ce que** le coussinet chauffant a une forme rectangulaire allongée et le coussinet chauffant comportant au moins deux cellules chauffantes en spirale disposées l'une derrière l'autre dans la direction longitudinale et les cellules chauffantes en spirale étant conçues comme des spirales de Fermat reliées entre elles.

2. Coussinet chauffant selon la revendication 1, **caractérisé en ce qu'**au moins une cellule chauffante a la forme d'une spirale d'Archimède, d'une spirale de Fermat, d'une triskèle ou d'une spirale multiple.

3. Coussinet chauffant selon la revendication 2, **caractérisé en ce que** la spirale a une forme ovale ou angulaire.

4. Coussinet chauffant selon l'une au moins des revendications précédentes, **caractérisé en ce que** la surface est au moins partiellement pourvue d'une couche adhésive.

5. Coussinet chauffant selon l'une au moins des revendications précédentes, **caractérisé en ce que** la cellule chauffante comporte un mélange de substances générant de la chaleur, une quantité définie de mélange de substances générant de la chaleur étant complètement enfermée par deux films polymères reliés l'un à l'autre, au moins un film polymère étant perméable à l'oxygène.

6. Coussinet chauffant selon l'une au moins des revendications précédentes, **caractérisé en ce que** la cellule chauffante comporte un mélange de substances générant de la chaleur, une quantité définie de mélange de substances générant de la chaleur étant enfermée dans un tube ou un tuyau, le tube ou le tuyau étant en un matériau perméable à l'oxygène.

7. Ceinture chauffante, comportant au moins un coussinet chauffant selon l'une au moins des revendications précédentes, **caractérisée en ce que** le coussinet chauffant permet une liaison en forme de boucle ou enveloppant le corps, au moyen d'éléments de ceinture qui sont fixés au niveau des axes longitudinaux et qui se terminent par un système de fermeture correspondant.

8. Ceinture chauffante selon la revendication 8, **caractérisée en ce que** les cellules chauffantes sont remplaçables.

9. Coussinet chauffant selon l'une au moins des revendications précédentes, **caractérisé en ce que** les mélanges de substances générant de la chaleur d'au moins une cellule chauffante comportent des matériaux à changement d'état (PCM) micro-encapsulés.

10. Coussinet chauffant selon l'une au moins des revendications précédentes, **caractérisé en ce qu'**une couche semi-convexe, se présentant sous la forme de lentille et formée à partir de polymères thermoconducteurs, est appliquée sur le côté face à la peau du coussinet chauffant au niveau des cellules chauffantes.
